# FASCICULE DE BREVET EUROPEEN

(11) **EP 0 870 748 B1**
(45) Date de publication et mention de la délivrance du brevet: **06.09.2000**
(21) Numéro de dépôt: 98400741.9
(22) Date de dépôt: 30.03.1998
(51) Int. Cl.: C07C 17/38, C07C 17/383, C07C 25/02, C07C 25/10, C07C 17/12

(54) **Procédé de purification d'hydrocarbures aromatiques chlorés**
Verfahren zur Reinigung von chlorierten aromatischen Kohlenwasserstoffen
Process for the purification of chlorinated aromatic compounds

(30) Priorité: 10.04.1997 FR 9704408
(43) Date de publication de la demande: 14.10.1998
(73) Titulaire: Atofina, 92800 Puteaux (FR)
(72) Inventeur: Commandeur, Raymond, 38220 Vizille (FR); Audoux, Dominique, 38360 Sassenage (FR)

(56) Documents cités:
- FR-A- 2 144 709

## Description

La présente invention concerne un procédé de purification des hydrocarbures aromatiques chlorés tels que notamment les trichlorobenzènes et dichlorotoluènes obtenus par chloration d'hydrocarbures aromatiques en présence de chlorure ferrique.

Les composés chlorés aromatiques ont une grande importance industrielle. Ils sont utilisés comme intermédiaires notamment pour la fabrication de produits agrochimiques, de colorants et de liquides diélectriques ininflammables.

Il existe de nombreuses méthodes permettant d'introduire un ou plusieurs atomes de chlore sur un noyau aromatique mais la plupart des procédés industriels utilisent la chloration par le chlore, catalysée par des chlorures métalliques. Les catalyseurs utilisables pour cette réaction sont AlCl₃, TiCl₄, SnCl₄, SbCl₃, SbCl₅, ZrCl₄, FeCl₃, NbCl₅, MoCl₅, WCl₆, GaCl₃, TeCl₄. Les plus généralement utilisés sont FeCl₃, AlCl₃, SbCl₃ et SbCl₅.

La réaction de chloration s'effectue généralement par introduction de chlore gazeux dans un réacteur contenant l'hydrocarbure aromatique à chlorer et le catalyseur ou système catalytique. La température de chloration est généralement comprise entre 40°C et 120°C.

On opère le plus souvent à pression atmosphérique. La réaction de chloration terminée, le milieu réactionnel ci-après désigné par brut réactionnel est soumis à un dégazage sous gaz inerte permettant de faire passer la teneur en HCl de quelques milliers de ppm à quelques centaines de ppm.

Ensuite, on effectue sur le brut réactionnel dégazé une opération de lavage à l'eau, éventuellement alcaline qui permet d'éliminer le catalyseur. Cette opération présente cependant de nombreux inconvénients, car il est nécessaire d'effectuer notamment le séchage du brut réactionnel lavé. En outre, cette opération de lavage génère des effluents contenant notamment des chlorures métalliques et des composés aromatiques chlorés ou organochlorés, ces derniers étant entraînés au moment dudit lavage à cause de leur solubilité dans l'eau. Ainsi, par exemple la solubilité dans l'eau du monochlorobenzène est de 150 ppm, celle des dichlorobenzènes est de 70 ppm et celle des trichlorobenzènes est de 20 ppm.

Ces organochlorés peuvent également être entraînés mécaniquement, sous forme de vésicules.

Compte tenu des réglementations en vigueur concernant le rejet des effluents industriels, il est impératif de traiter ces rejets aqueux pour abaisser leur teneur en organochlorés de même qu'en chlorures métalliques.

Ces opérations de traitement sont coûteuses et peu de solutions efficaces ont été proposées.

Ainsi, dans le brevet US 4,885,418, on propose d'effectuer le passage d'un milieu réactionnel provenant de la chloration d'hydrocarbures aromatiques en présence de chlorures métalliques sur une résine anionique humide sous forme hydroxyle.

Bien que ce procédé présente l'avantage de pouvoir éliminer la majeure partie des chlorures métalliques, il présente cependant de nombreux inconvénients. En effet, il est nécessaire d'effectuer un dégazage très poussé du milieu réactionnel pour éliminer l'acide chlorhydrique avant passage sur la résine anionique. Ensuite, la régénération des résines est coûteuse et nécessite l'emploi de solutions aqueuses d'acide chlorhydrique puis de soude et conduit inévitablement à des effluents aqueux qu'il faut traiter. En outre, ce procédé est totalement silencieux sur l'élimination des organochlorés.

On a maintenant trouvé un procédé de purification par distillation d'hydrocarbures aromatiques chlorés obtenus par chloration d'hydrocarbures aromatiques non chlorés ou partiellement chlorés en présence de chlorure ferrique FeCl₃ ; ledit procédé étant caractérisé en ce que avant la distillation desdits hydrocarbures aromatiques chlorés on effectue un dégazage partiel du milieu réactionnel ou brut réactionnel et que l'on soumet directement ce brut réactionnel dégazé à une distillation en présence du FeCl₃.

A titre d'illustration d'hydrocarbures aromatiques non chlorés ou partiellement chlorés désignés ci-après par hydrocarbures aromatiques, on citera le toluène, les mono- et dichlorotoluènes, les paraxylènes, les chloroxylènes, l'orthodichlorobenzène, les trichlorobenzènes.

Le procédé de chloration est connu et peut être effectué généralement par introduction du chlore gazeux dans l'hydrocarbure aromatique à chlorer contenant le FeCl₃. L'opération de chloration s'effectue généralement à pression atmosphérique à une température comprise entre 40°C et 120°C et, de préférence, entre 60°C et 120°C.

Selon la présente invention, le brut réactionnel provenant de la réaction de chloration d'hydrocarbures aromatiques en présence de FeCl₃ est dégazé partiellement sous courant de gaz inerte tel que l'azote, à une température qui est avantageusement celle de la chloration ou à une température inférieure et, de préférence, sous pression atmosphérique.

Selon la présente invention, la distillation des hydrocarbures aromatiques chlorés s'effectue en présence du FeCl₃ utilisé, dans la réaction de chloration, en une quantité qui peut aller de 10 à 300 ppm et, de préférence, entre 50 ppm et 200 ppm. On ne sortirait pas du cadre de l'invention si on associe au FeCl₃ un cocatalyseur contenant du soufre tel que les dérivés N-substitués de la phénothiazine (N-chloracarbonylphénothiazine).

Selon la présente invention, la distillation du brut réactionnel dégazé - distillation qui s'effectue sans que le FeCl₃ ou système catalytique ne soit éliminé dudit brut réactionnel dégazé - peut être réalisé soit en discontinu, soit en continu.

Dans le cas où elle est réalisée en discontinu, le brut réactionnel dégazé contenant le FeCl₃ est chargé directement dans un bouilleur d'une colonne à distiller. Il distille en tête de colonne un mélange d'hydrocarbures aromatiques non transformés ainsi que de l'acide chlorhydrique résiduel puis ensuite les hydrocarbures aromatiques chlorés. Le résidu est constitué de chlorures de fer et d'organochlorés lourds.

Lorsque la distillation est réalisée en continu, on alimente avec le brut réactionnel dégazé contenant le FeCl₃ une unité de distillation constituée de plusieurs colonnes.

Selon un mode préféré, on utilise une unité de distillation constituée de deux colonnes.

Avec le brut réactionnel dégazé contenant un mélange constitué d'hydrocarbures aromatiques chlorés, d'hydrocarbures aromatiques de départ non transformés, le FeCl₃ et des traces d'HCl on alimente une première colonne remplie par un garnissage.

De cette première colonne il distille en tête un mélange constitué des réactifs de départ non transformés et d'acide chlorhydrique. Ce mélange peut être recyclé au réacteur de chloration.

En pied de colonne, on obtient un mélange comprenant les produits aromatiques chlorés et les chlorures de fer.

Avec ce mélange, on alimente une seconde colonne pouvant présenter les mêmes caractéristiques que la première colonne.

On opère cependant de préférence avec des conditions de température et de pression différentes, qui dépendent des caractéristiques physico-chimiques des produits chlorés obtenus.

On distille en tête de colonne les produits chlorés éventuellement sous forme de mélange d'isomères ou tels quels et en pied de colonne on a des produits surchlorés non recherchés et les chlorures de fer ; ce pied, pouvant être extrait en continu ou en discontinu, est ensuite éliminé notamment par incinération.

Ce procédé s'applique tout particulièrement à la préparation des chlorotoluènes et des trichlorobenzènes obtenus par chloration, en présence de FeCl₃, respectivement du toluène et des dichlorobenzènes.

On obtient des produits incolores, avec une très faible teneur en HCl et en FeCl₃.

En outre, on constate après plusieurs mois voire plusieurs années de fonctionnement aucune corrosion des appareillages de distillation qui sont en matériaux simples tels que l'acier et seulement un encrassement très faible des colonnes ce qui permet de conserver l'efficacité de la séparation des produits ayant des points d'ébullition relativement proches.

Cet encrassement peut avantageusement être éliminé par un simple traitement à l'eau de la colonne considérée.

En outre, on constate que la distillation du brut réactionnel dégazé en présence du catalyseur de chloration ne modifie par le jeu d'une réaction d'isomérisation de la composition isomérique soumise à ladite distillation.

Les exemples qui suivent illustrent l'invention.

### EXEMPLE 1

### DICHLOROTOLUENES OBTENUS PAR CHLORATION CONTINUE DU TOLUENE EN PRESENCE DE FECL₃

Le brut réactionnel provenant de l'opération d'une chloration du toluène en présence de FeCl₃ est soumis à un dégazage partiel à l'azote à une température comprise entre 80°C et 90°C, sous pression atmosphérique.

Ce brut réactionnel partiellement dégazé a la composition suivante (les pourcentages sont exprimés en poids) :
ortho et parachlorotoluène : 80,7 %,
2,3-, 2,4-, 2,5-, 2,6-, 3,4-dichlorotoluènes : 18,6 %
trichlorotoluènes : 0,7 %,
HCl : 400 ppm et FeCl₃ : 400 ppm

On alimente en continu avec ce brut réactionnel une colonne à distiller chauffée au moyen de vapeur 6 bars et remplie par un garnissage SULZER correspondant à une efficacité de 15 plateaux réels. L'alimentation s'effectue en milieu de colonne avec un débit de 2350 kg/h. Le taux de reflux est de 0,7.

La distillation s'effectue sous une pression de 8000 Pa (60 mm de Hg). La température de pied est de 122°C et la température de tête est de 76°C.

Il distille en tête un liquide contenant les ortho- et parachlorotoluènes et des traces de HCl. Ce mélange est recyclé dans le réacteur de chloration.

Le liquide en pied de colonne est constitué par :
- le mélange des dichlorotoluènes mentionné ci-avant,
- les trichlorotoluènes,
- les chlorures de fer (FeCl₃ et FeCl₂)

Avec ce mélange, on alimente en continu un bouilleur de 18 m³ d'une colonne à distiller constituée de 30 plateaux à cloches (15 plateaux réels) fonctionnant avec de la vapeur 14 bars et sous une pression de 3,333 Pa (25 mm de mercure). Le taux de reflux est de 1.

La température de tête est d'environ 100°C

La température de pied bouilleur est de 160 à 180°C.

On obtient en tête de colonne un liquide incolore qui a la composition suivante :
- monochlorotoluène : 0,4 %
- 2,5-dichlorotoluène : 26,4 %
- 2,6-dichlorotoluène : 21,3 %
- 2,4-dichlorotoluène : 36 %
- 2,4-dichlorotoluène : 5,8 %
- 2,3-dichlorotoluène : 10 %
- trichlorotoluènes : 0,1 %

Teneur en HCl :2,1 ppm

Teneur en chlore hydrolysable : 1,7 ppm (chlore aliphatique).

Ce produit liquide convient parfaitement dans l'emploi comme solvant ou comme liquide diélectrique.

Le produit obtenu en pied de colonne constitué essentiellement par des trichlorotoluènes et des chlorures de fer s'accumule progressivement dans le bouilleur et en est extrait en semi-continu sans que cela pose un quelconque problème d'extraction ni même d'encrassement dudit bouilleur.

L'extrait est détruit par incinération dans un centre agréé pour la destruction des résidus chlorés.

Après de nombreuses années de fonctionnement, on n'observe aucun problème de corrosion des matériaux constituant l'unité de distillation qui est en acier ordinaire.

On observe seulement une légère diminution de l'efficacité de la première colonne au bout d'environ 3 à 4 mois. Cette efficacité est retrouvée rapidement si l'on effectue un simple lavage à l'eau à reflux d'une durée égale à environ 24 heures.

### EXEMPLE 2

### PURIFICATION DE TRICHLOROBENZENES OBTENUS PAR CHLORATION D'ORTHODICHLOROBENZENE EN PRESENCE DE FECL₃

Le brut réactionnel provenant de l'opération chloration d'orthodichlorobenzène en présence de FeCl₃ est soumis à un léger dégazage à l'azote. Ce dégazage est effectué dans un simple bac, avec un débit d'azote égal à 25 m³/h, à une température comprise entre 75°C et 80°C, sous pression atmosphérique. Ce brut réactionnel dégazé a la composition suivante : (les pourcentages sont exprimées en poids) :
- orthodichlorobenzène : 75 %
- 1,2,3- et 1,2,4-trichlorobenzènes : 24 %
- tetrachlorobenzènes : 1 %
- HCl :200 ppm
- FeCl₃ : 200 ppm

On alimente en continu avec ce brut réactionnel dégazé une colonne à distiller chauffée au moyen de vapeur à 190°C et remplie par un garnissage structuré du type SULZER (tôles gaufrées soudées par point) correspondant à une efficacité de 25 plateaux réels.

L'alimentation s'effectue en milieu de colonne avec un débit de 2400 kg/h. Le taux de reflux est de 1.

La distillation s'effectue sous une pression de 18.666 Pa (140 mm de Hg).

La température de pied de colonne est de 170°C et la température de tête de colonne est de 120°C.

Il distille en tête un liquide constitué par l'orthochlorobenzène (non-transformé) contenant un peu de HCl. Ce liquide est recyclé dans le réacteur de chloration.

Le liquide en pied de colonne est constitué par
- un mélange des isomères de trichlorobenzènes,
- des tetrachlorobenzènes,
- du pentachlorobenzène,
- des chlorures de fer (FeCl₃, FeCl₂).

Avec ce mélange, on alimente en continu une seconde colonne à distiller ayant le même garnissage et les mêmes caractéristiques que la première colonne (efficacité égale à 25 plateaux théoriques).

On alimente cette colonne en milieu de colonne dont le chauffage est assuré par de la vapeur à 190°C. On distille sous une pression de 6,666 Pa (50 mm de Hg). La température en tête de colonne est de 120°C et en pied de colonne de 160°C.

On obtient en tête de colonne, avec un débit de 600kg/h un liquide incolore qui a la composition suivante :
- orthodichlorobenzène : 0,15 %
- 1,2,3- et et 1,2,4-trichlorobenzènes : 99,84 %
- tetrachlorobenzènes : 0,007 %
- HCl < 10 ppm

Ce mélange de trichlorobenzènes est utilisable comme solvant, liquide diélectrique ininflammable ou comme matière première de base pour l'obtention du 1,2,3-trichlorobenzène pur et du 1,2,4-trichlorobenzène pur.

Le résidu obtenu en pied de colonne est constitué par un mélange des tétrachlorobenzènes, du pentachlorobenzène et de chlorures de fer. Ce résidu est extrait en continu (2,5 kg/h) et est ensuite, comme dans l'exemple 1, incinéré dans un centre agréé pour la destruction des résidus chlorés.

Là encore, après de nombreux mois de fonctionnement on n'observe aucune corrosion des matériaux constituant les 2 colonnes de distillation.

### EXEMPLE 3

On réalise un essai comparatif qui consiste à comparer l'efficacité de notre procédé lorsque celui-ci est appliqué à un mélange de trichlorobenzènes contenant du FeCl₃ (conformément à l'invention) ou bien AlCl₃ ou SbCl₃ (non conforme à l'invention).

On distille 800 g d'un mélange constitué par (les pourcentages sont exprimés en poids) :
- orthodichlolrobenzène : 1,25 %
- 1,2,4-trichlorobenzène: 82,35 %
- 1,2,3-trichlorobenzène : 14,30 %
- 1,2,4,6-tetrachlorobenzène: 0,5 %
- 1,2,3,4-tetrachlorobenzène : 0,6 %
- catalyseur ( FeCl₃, AlCl₃ ou SbCl₃) : 1 %

Ce mélange est introduit dans un ballon en verre surmonté d'une colonne adiabatique de 1,5 mètres remplie par un garnissage à base de ressorts en verre (7 plateaux réels).

La distillation est réalisée en continu sous une pression de 6,666 Pa (50 mm de Hg) avec un taux de reflux de 1. Le chauffage est assuré par un chauffe ballon, la distillation est régularisée par introduction d'azote au moyen d'un tube capillaire.

On réalise trois fractions F1, F2, F3 de 200 g et on laisse en pied 200 g.

La température de tête est de 120-123°C, alors que la température de pied est de 130°C. On reporte dans le tableau 1 ci-après l'analyse des fractions.

Dans ce tableau, on reporte :
- la couleur des fractions,
- la teneur en métal correspondant au catalyseur,
- la teneur pondérale en Cl⁻ (soit sous forme HCl, soit sous forme de chlorures).

Nous n'avons pas reporté les résultats pour AlCl₃ car la distillaiton est impossible, il y a de nombreux bouchages en tête de colonne.

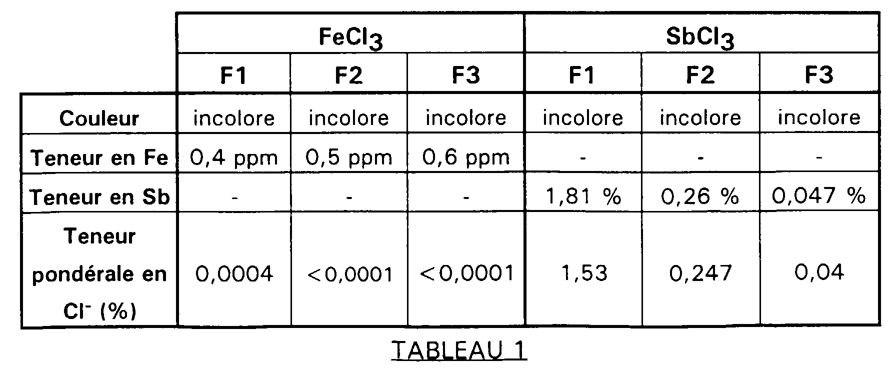

On constate que FeCl₃ et SbCl₃ bien qu'ayant des points d'ébullition supérieurs aux hydrocarbures aromatiques chlorés, seul FeCl₃ reste en pied de colonne.

## Revendications

1. Procédé de purification par distillation d'hydrocarbures aromatiques chlorés obtenus par chloration d'hydrocarbures aromatiques non chlorés ou partiellement chlorés en présence de chlorure ferrique FeCl₃ ; ledit procédé étant caractérisé en ce que avant la distillation desdits hydrocarbures aromatiques chlorés on effectue un dégazage partiel du brut réactionnel et que l'on soumet directement ce brut réactionnel dégazé à une distillation en présence du FeCl₃.

2. Procédé selon la revendication 1, caractérisé en ce que l'hydrocarbure aromatique non chloré est le toluène.

3. Procédé selon la revendication 1, caractérisé en ce que l'hydrocarbure aromatique partiellement chloré est l'orthodichlorobenzène.

4. Procédé selon la revendicaiton 1, caractérisé en ce que les hydrocarbures aromatiques chlorés soumis à la purification par distillation sont un mélange comprenant des dichlorotoluènes et du FeCl₃ provenant de la chloration du toluène en présence de FeCl₃.

5. Procédé selon la revendication 1, caractérisé en ce que les hydrocarbures aromatiques chlorés soumis à la purification par distillation sont un mélange comprenant des trichlorobenzènes et du FeCl₃ provenant de la chloration de l'orthodichlorobenzène en présende de FeCl₃.

## Patentansprüche

1. Verfahren zur destillativen Reinigung chlorierter aromatischer Kohlenwasserstoffe, die durch Chlorierung nichtchlorierter oder partiell chlorierter aromatischer Kohlenwasserstoffe in Gegenwart von Eisenchlorid FeCl₃ erhalten sind, dadurch gekennzeichnet, daß man vor der Destillation dieser chlorierten aromatischen Kohlenwasserstoffe eine teilweise Entgasung der Reaktionsrohmischung vornimmt und man die entgaste Reaktionsrohmischung direkt einer Destillation in Gegenwart von FeCl₃ unterwirft.

2. Verfahren nach Anspruch 1, dadurch gekennzeichnet, daß der nichtchlorierte aromatische Kohlenwasserstoff Toluol ist.

3. Verfahren nach Anspruch 1, dadurch gekennzeichnet, daß der partiell chlorierte aromatische Kohlenwasserstoff ortho-Dichlorbenzol ist.

4. Verfahren nach Anspruch 1, dadurch gekennzeichnet, daß die der destillativen Reinigung unterworfenen, chlorierten aromatischen Kohlenwasserstoffe eine Mischung sind, welche Dichlortoluole und FeCl₃ umfaßt, die aus der Chlorierung des Toluols in Gegenwart von FeCl₃ stammen.

5. Verfahren nach Anspruch 1, dadurch gekennzeichnet, daß die der destillativen Reinigung unterworfenen, chlorierten aromatischen Kohlenwasserstoffe eine Mischung sind, welche Trichlorbenzole und FeCl₃ umfaßt, die aus der Chlorierung von ortho-Dichlorbenzol in Gegenwart von FeCl₃ stammen.

## Claims

1. Process for the purification by distillation of chlorinated aromatic hydrocarbons obtained by chlorination of non-chlorinated or partially chlorinated aromatic hydrocarbons in the presence of ferric chloride FeCl₃; the said process being characterized in that, before the distillation of the said chlorinated aromatic hydrocarbons, the reaction crude is partially degassed and that this degassed reaction crude is directly distilled in the presence of the FeCl₃.

2. Process according to Claim 1, characterized in that the non-chlorinated aromatic hydrocarbon is toluene.

3. Process according to Claim 1, characterized in that the partially chlorinated aromatic hydrocarbon is ortho-dichlorobenzene.

4. Process according to Claim 1, characterized in that the chlorinated aromatic hydrocarbons purified by distillation are a mixture comprising dichlorotoluenes and FeCl₃ originating from the chlorination of toluene in the presence of FeCl₃.

5. Process according to Claim 1, characterized in that the chlorinated aromatic hydrocarbons purified by distillation are a mixture comprising trichlorobenzenes and FeCl₃ originating from the chlorination of ortho-dichlorobenzene in the presence of FeCl₃.
